# FASCICULE DE BREVET EUROPEEN

(11) **EP 3 809 132 B1**
(45) Date de publication et mention de la délivrance du brevet: **01.02.2023**
(21) Numéro de dépôt: 20201599.6
(22) Date de dépôt: 13.10.2020
(51) Int. Cl.: G01N 33/22, C10K 1/04, G01N 33/00, C10J 3/84

(54) **PROCEDE D'ANALYSE IN SITU DE GOUDRONS DANS UN GAZ**
VERFAHREN ZUR IN-SITU-ANALYSE VON TEER IN EINEM GAS
METHOD FOR IN-SITU ANALYSIS OF TAR IN A GAS

(30) Priorité: 15.10.2019 FR 1911480
(43) Date de publication de la demande: 21.04.2021
(73) Titulaire: Commissariat à l'énergie atomique et aux énergies alternatives, 75015 Paris (FR)
(72) Inventeur: RAVEL, Serge, 38054 Grenoble Cedex 09 (FR)
(74) Mandataire: Brevalex

(56) Documents cités:
- FR-A1- 3 011 556
- M SIMONE ET AL: "Biomass Tar: Assessment of a Sampling Device and a Characterization Methodology", PROCEEDINGS OF THE EUROPEAN BIOMASS CONFERENCE AND EXHIBITION.BIOMASS FOR ENERGY, INDUSTRY AND CLIMATE PROTECTION 19TH CONF., juin 2011 (2011-06), XP055711670, DOI: 10.5071/19thEUBCE2011-VP2.3.21 ISBN: 978-88-89407-55-4
- W. M.T.M. Reimerink: "The use of activated carbon as catalyst and catalyst carrier in industrial applications" In: "Studies in surface science and catalysis Vol 120 Part A", 1999, Elsevier Science B.V., XP055711523, ISBN: 978-0-444-50165-3 pages 751-769, DOI: 10.1016/S0167-2991(99)80571-0, * page 761 *

## Description

### DOMAINE TECHNIQUE

La présente invention se rapporte au domaine général de l'analyse des gaz, par exemple, issus des procédés de pyrolyse et/ou de gazéification de la biomasse.

L'invention concerne un procédé d'évaluation de la quantité de goudrons dans un gaz, en particulier dans un syngas.

L'invention concerne également un dispositif permettant la mise en oeuvre de ce procédé.

L'invention est particulièrement intéressante puisqu'elle permet de évaluer rapidement *in situ* et en semi-continu la quantité de goudrons présents dans un gaz.

### ÉTAT DE LA TECHNIQUE ANTÉRIEURE

La valorisation de la biomasse en biocombustibles/biocarburants aptes à remplacer les combustibles/carburants d'origine fossile est devenue un enjeu majeur. La plupart des procédés de transformation thermochimique de la biomasse comprennent une étape de pyrolyse et une étape de gazéification de la biomasse à la vapeur d'eau pour obtenir un gaz de synthèse composé essentiellement de monoxyde de carbone (CO), de dihydrogène (H₂), de dioxyde de carbone (CO₂) et, pour les procédés autothermiques à l'air, de l'azote (N₂). A partir de CO et d'H₂, il est alors possible d'obtenir des chaînes d'hydrocarbures CH₂ semblables à celles provenant des hydrocarbures d'origine fossile et ainsi fabriquer un carburant de synthèse.

Cependant, dans les gaz issus de procédé de gazéification, lors de l'étape de pyrolyse qui précède l'étape de gazéification, se forment des polluants organiques, et notamment des goudrons (ou « tar » en terme anglo-saxon). Il s'agit d'une phase dite lourde comprenant des molécules hydrocarbonées condensables, aliphatique ou ayant un ou plusieurs cycles aromatiques et, par exemple, pouvant atteindre une masse molaire supérieure à 100g/mol. En particulier, dans les goudrons, on retrouve les hydrocarbures aromatiques polycycliques (ou HAP) tel que le naphtalène, l'anthracène, etc.

Ces polluants présentent le défaut de se condenser dans les tuyauteries de sorties des gazéifieurs dès que le gaz est à une température inférieure à 250-300°C, ce qui conduit au colmatage de la ligne et diminue les rendements.

Les problèmes induits par ce phénomène dépendent de la concentration et de la composition de ces goudrons. Selon les technologies et les ressources gazéifiées, il se forme de quelques mg/Nm³ à quelques centaines de g/Nm³ de goudrons dans les gaz.

Il est donc essentiel de connaître la quantité de goudrons dans les gaz de synthèse brut (syngas) afin de déterminer les mesures à prendre (nettoyage, etc).

Actuellement, plusieurs techniques de dosage des goudrons dans les gaz existent. La plus connue consiste à prélever un échantillon de gaz chaud (typiquement à une température de 250 °C) puis de le faire barboter dans un jeu de barboteurs remplis de solvant dont la température descend jusqu'à une température finale de -20 °C, température à laquelle les goudrons sont absorbés dans le solvant [1]. Cependant cette technique est longue à mettre en place, puisque, d'une part, le gaz doit barboter suffisamment longtemps et, d'autre part, l'analyse de la solution est effectuée en différé par Chromatographie en phase gazeuse avec détection par ionisation de flamme (GCFiD) ou spectrométrie de masse (MS) qui est une méthode d'analyse relativement longue. Donc, entre le prélèvement de l'échantillon de gaz et l'obtention des résultats, plusieurs heures sont nécessaires. De plus de telles méthodes utilisent des solvants, ce qui nécessite un traitement particulier des effluents.

Pour s'affranchir de cet inconvénient, une autre technique basée sur l'adsorption des goudrons sur des phases solides (SPA) suivie d'une désorption thermique (TD) a été étudiée [2]. Les limites de détection sont satisfaisantes. Cependant, l'étape de désorption peut être difficile à maitriser, et une analyse par chromatographie en phase gazeuse (GCFiD ou MS) est aussi utilisée. De plus, cette technique donne des bons résultats pour les faibles concentrations (de l'ordre de quelques mg/Nm³) mais n'est pas adaptée aux concentrations élevées (supérieures à quelques g/Nm³).

Ces méthodes d'analyses nécessitent des appareils coûteux, du personnel qualifié et les résultats sont assez longs à obtenir, ce qui rend le procédé difficilement industrialisable et coûteux.

Plus récemment, des méthodes d'analyses dites en ligne ont vu le jour. Parmi ces méthodes, on peut citer la détection et quantification de goudrons dans une cellule de mesure équipée de diodes électroluminescentes émettant à différentes longueurs d'onde [3], par mesure directe par spectrométrie de masse à faible énergie d'ionisation (IMR GS) [4], par détection par photo-ionisation (PID) [5], par analyse par fluorescence induite par laser [6], par spectrométrie de masse à faisceau moléculaire (MBMS) [7], ou encore par spectrométrie à temps de vol (TOFMS) [8].

On notera également les documents suivants : M SIMONE ET AL: "Biomass Tar: Assessment of a Sampling Device and a Characterization Methodology",PROCEEDINGS OF THE EUROPEAN BIOMASS CONFERENCE AND EXHIBITION.BIOMASS FOR ENERGY, INDUSTRY AND CLIMATE PROTECTION 19TH CONF., juin 2011 (2011-06), XP055711670,DOI: 10.5071/19thEUBCE2011-VP2.3.21ISBN: 978-88-89407-55-4 et FR3011556A1.

Toutes ces méthodes d'analyse donnent une composition détaillée des goudrons. Cependant, elles sont assez complexes à mettre en oeuvre et/ou ne sont utilisables que pour une certaine gamme de concentration en goudrons dans le gaz.

### EXPOSÉ DE L'INVENTION

Un but de la présente invention est de proposer un procédé d'analyse *in situ* permettant d'obtenir rapidement la quantité de goudrons, ou au moins un ordre de grandeur de la quantité de goudrons, présente dans un gaz, tout en étant simple à mettre en oeuvre.

Pour cela, la présente invention propose un procédé d'évaluation de la quantité de goudrons dans un gaz, en particulier dans un syngas, comprenant les étapes successives suivantes :
a) fourniture d'un dispositif comprenant :
   - une capacité comprenant une entrée de gaz, raccordée à une ligne d'arrivée d'un gaz contenant des goudrons, une sortie de gaz, et une entrée de dioxygène,
   - un appareil de mesure de CO₂, raccordé à la sortie de gaz,
   - un système de refroidissement,
   - un système de chauffage,
   - un dispositif d'ignition pour amorcer la combustion de vapeurs de goudrons,
b) refroidissement de la capacité à une température inférieure à -15°C, et de préférence inférieure à -20°C, avec le système de refroidissement,
c) circulation du gaz à travers la capacité, depuis l'entrée de gaz jusqu'à la sortie de gaz, les goudrons se condensant dans la capacité, moyennant quoi on obtient des goudrons condensés, puis arrêt de la circulation de gaz,
d) chauffage de la capacité, à une température supérieure à 250°C, et de préférence supérieure à 300°C, avec le système de chauffage, de manière à vaporiser les goudrons condensés dans la capacité 100, moyennant quoi on forme des vapeurs de goudrons,
e) introduction du dioxygène dans la capacité, par l'entrée de dioxygène, amorçage et combustion des vapeurs de goudrons, moyennant quoi on forme des gaz de combustion puis arrêt de l'introduction de dioxygène,
f) évacuation des gaz de combustion par la sortie de gaz jusqu'à l'appareil de mesure et mesure la quantité de CO₂ dans les gaz de combustion, puis
g) détermination la quantité de goudrons condensés puis brûlés à partir de la quantité de CO₂ mesurée lors de l'étape f).

L'invention se distingue fondamentalement de l'art antérieur par la détermination de la quantité de goudrons initialement présente dans le gaz à partir de la quantité de dioxyde de carbone produite lors de la combustion de ces goudrons. L'invention repose sur la condensation des goudrons et leur capacité à brûler. Avec un tel procédé, il est possible de déterminer rapidement *in situ* et en semi-continu la quantité de goudrons présente dans un gaz.

Un tel procédé peut être utilisé pour des gaz :
- peu chargés en goudrons (typiquement pour des concentrations massiques de l'ordre de 0,1g/Nm³),
- légèrement chargés en goudrons (typiquement pour des concentrations massiques de l'ordre de 1g/Nm³),
- chargés en goudrons (typiquement pour des concentrations massiques de l'ordre de 10g/Nm³), ou
- très chargés en goudrons (typiquement pour des concentrations massiques de l'ordre de 100g/Nm³).

Avantageusement, la capacité comprend un solide poreux, par exemple une mousse métallique pour augmenter la surface d'échange et/ou faciliter le piégeage des goudrons dans la capacité.

Avantageusement, un piège barboteur est disposé entre la capacité et l'appareil de mesure de CO₂. Ce piège permet, par exemple, de vérifier la présence de goudrons en sortie de la capacité et éventuellement de déterminer si les étapes d) à g) doivent être réalisées. Il peut également servir à piéger l'eau formée durant l'étape de combustion des goudrons.

Avantageusement, le dispositif comprend en outre un régulateur de débit massique pour contrôler le débit massique de gaz circulant à travers la capacité lors de l'étape c).

Avantageusement, le système de refroidissement et/ou le système de chauffage sont enroulés autour de la capacité. Par exemple, le chauffage est assuré par un chauffant électrique enroulé autour de la capacité. Le chauffage électrique est rapide et présente peu d'inertie. Le refroidissement peut être assuré par un fluide qui circule dans un tuyau enroulé également autour de la capacité. Le refroidissement par un fluide est simple à mettre en oeuvre en utilisant un groupe froid externe.

Avantageusement, la capacité comprend une partie centrale tubulaire disposée entre une partie supérieure et une partie inférieure. La partie supérieure est, de préférence, conique, notamment pour faciliter l'introduction et l'homogénéisation du gaz dans la capacité.

Par partie supérieure, on entend la partie en amont de la capacité. Par partie inférieure, on entend la partie en aval de la capacité. Ces termes sont utilisés en tenant compte du sens de circulation du gaz à travers la capacité.

Avantageusement, le système de refroidissement est enroulé autour de la partie centrale de la capacité de manière à faire condenser les goudrons au centre de la capacité et ainsi créer un gradient de température le long de la partie supérieure pour éviter la condensation trop rapide des goudrons en entrée, ce qui pourrait empêcher la circulation.

Avantageusement, le système de chauffage est disposé autour de la partie centrale et autour de la partie supérieure de la capacité.

Selon un mode de réalisation particulièrement avantageux, le système de chauffage comprend une première partie disposée autour de la partie supérieure et une deuxième partie disposée autour de la partie centrale de la capacité. La première partie et la deuxième partie peuvent fonctionner indépendamment l'une de l'autre. Par exemple, la première partie peut fonctionner lors de l'étape c) pour éviter la condensation des gaz au niveau de l'entrée de la capacité. Les gaz seront condensés dans la partie centrale de la capacité. La deuxième partie peut fonctionner lors de l'étape d).

Avantageusement, l'étape d) et/ou l'étape e) et/ou l'étape f) sont suivies en mesurant la pression dans la capacité.

L'invention concerne également un dispositif comprenant :
- une capacité comprenant une entrée de gaz, une sortie de gaz, une entrée de dioxygène, un dispositif d'ignition pour amorcer la combustion de vapeurs de goudrons,
- un appareil de mesure de CO₂, raccordé à la sortie de gaz, et permettant de mesurer la quantité de CO₂,
- un système de refroidissement, configuré pour refroidir la capacité jusqu'à des températures inférieures à -15°C, et de préférence inférieures à -20°C,
- un système de chauffage, configuré pour chauffer la capacité jusqu'à des températures supérieures à 250°C, et de préférence supérieures à 300°C.

D'autres caractéristiques et avantages de l'invention ressortiront du complément de description qui suit.

Il va de soi que ce complément de description n'est donné qu'à titre d'illustration de l'objet de l'invention et ne doit en aucun cas être interprété comme une limitation de cet objet.

### BRÈVE DESCRIPTION DES DESSINS

La présente invention sera mieux comprise à la lecture de la description d'exemples de réalisation donnés à titre purement indicatif et nullement limitatif en faisant référence aux dessins annexés sur lesquels :
La figure 1 représente de manière schématique, en coupe, un dispositif pour analyser *in situ* en semi-continu la quantité de goudrons présente dans un gaz, selon un mode de réalisation particulier de l'invention,
Les figures 2A à 2E représentent de manière schématique différentes étapes du procédé d'analyse *in situ* de la quantité de goudrons présente dans un gaz, selon un mode de réalisation particulier de l'invention ; les vannes avec le symbole F sont des vannes fermées et les vannes avec le symbole O sont des vannes ouvertes.

Les différentes parties représentées sur les figures ne le sont pas nécessairement selon une échelle uniforme, pour rendre les figures plus lisibles.

Les différentes possibilités (variantes et modes de réalisation) doivent être comprises comme n'étant pas exclusives les unes des autres et peuvent se combiner entre elles.

En outre, dans la description ci-après, des termes qui dépendent de l'orientation du dispositif s'appliquent en considérant que la structure est orientée de façon illustrée sur les figures.

### EXPOSÉ DÉTAILLÉ DE MODES DE RÉALISATION PARTICULIERS

Par la suite on décrira plus particulièrement un procédé pour déterminer la concentration en goudrons d'un syngas. Cependant, l'invention est transposable à d'autres gaz ou à d'autres mélanges de gaz.

Par syngas, on entend un gaz de synthèse brut issu, par exemple d'un procédé de pyrolyse ou de gazéification d'une matière carbonée, et en particulier de la biomasse. Le syngas contient majoritairement les gaz suivants : CO, CO₂, H₂ et CH₄. Le syngas est pollué ou contaminé par des goudrons résultant de la combustion incomplète de la matière carbonée. Les goudrons comprennent des hydrocarbures aromatiques polycycliques (HAP), tel que l'anthracène, le naphtalène et le pyrène seuls ou en mélange. Ils peuvent contenir d'autres composés condensables qui dépendent de la nature du procédé de conversion thermochimique, en particulier de la température de traitement.

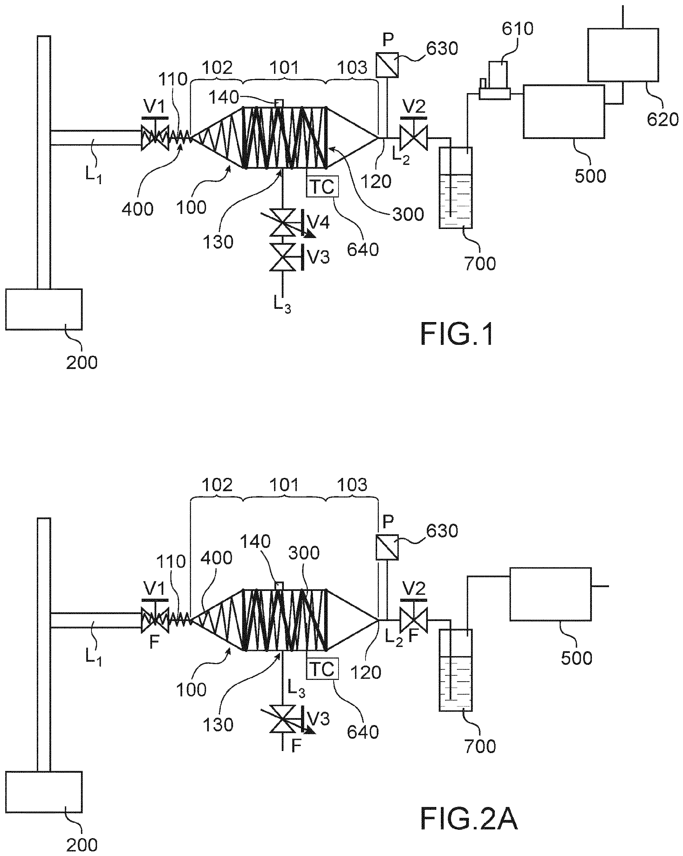

Le procédé met en oeuvre un dispositif comprenant une capacité 100 (figure 1). La capacité 100 est une chambre réactionnelle permettant, d'une part, de piéger les goudrons présents dans le syngas en les condensant et/ou adsorbant, et, d'autre part, de les vaporiser et de les brûler.

Le volume de la capacité peut être adapté en fonction de la quantité de goudrons à piéger.

La capacité 100 comprend une entrée de gaz 110, reliée par exemple à un réacteur de pyrolyse ou à un réacteur de gazéification 200 via une ligne dite de prélèvement L₁. Une première vanne V1 est disposée au niveau de l'entrée de gaz 110. Un filtre peut être disposé au niveau de la ligne de prélèvement pour filtrer le syngas avant d'être introduit dans la capacité 100.

La capacité 100 comprend une sortie de gaz 120 configurée pour évacuer le syngas dépourvu de goudrons après adsorption et/ou condensation de ces derniers et/ou les produits gazeux résultant de la combustion des goudrons vers une ligne d'analyse L₂. Une vanne V2 est disposée au niveau de la sortie de gaz 120.

La capacité 100 comprend également une entrée de dioxygène 130, raccordée à une ligne d'alimentation en dioxygène L₃. Il peut s'agir de dioxygène pur ou d'un mélange gazeux contenant du dioxygène tel que de l'air. Une troisième vanne V3 est disposée entre la capacité 100 et l'alimentation en dioxygène. Une vanne de laminage permettant de régler finement le débit d'air V4 peut également être disposée sur la ligne L₃.

La capacité 100 comprend également un dispositif d'ignition 140 pour apporter l'énergie d'ignition (ou énergie d'activation) de la combustion de vapeurs de goudrons.

Ce dispositif 140 est activé après l'introduction du dioxygène dans la capacité.

Par exemple, le dispositif d'ignition 140 peut permettre de former un arc électrique ou une flamme. Il peut comprendre un élément piézoélectrique ou un filament fusible.

Lorsque la première vanne V1, la deuxième vanne V2 et la troisième vanne V3 sont fermées, la capacité 100 forme une enceinte étanche.

La capacité 100 comprend, depuis l'entrée de gaz 110 vers la sortie de gaz 120 : une partie dite supérieure 102, une partie centrale 101 et une partie dite inférieure 103. De préférence la partie centrale 101 est tubulaire. De préférence, la partie supérieure 101 et, éventuellement, la partie inférieure 103 sont coniques. Une forme conique facilite l'homogénéisation des réactifs au sein de la capacité.

De préférence, la capacité 100 est métallique. On choisira un matériau résistant aux températures utilisées au cours du procédé, et de préférence, résistant à de nombreux cycles de refroidissement/chauffage.

De préférence, la capacité 100 contient un solide poreux permettant une augmentation de la surface d'échange et/ou d'adsorption. Il peut s'agir d'une mousse métallique. Des matériaux adsorbant les goudrons peuvent être disposés dans le solide poreux.

La capacité 100 est équipée d'un système de refroidissement 300.

Le système de refroidissement 300 peut être positionné à l'intérieur de la capacité 100.

De préférence, le système de refroidissement 300 est externe, i.e. positionnée à l'extérieur de la capacité 100. Le système de refroidissement 300 est configuré pour refroidir l'intérieur de la capacité jusqu'à des températures inférieures à - 15°C et de préférence inférieure à -20°C de manière à faire condenser les goudrons présents à l'intérieur de la capacité.

Le système de refroidissement 300 comprend, par exemple, des tubes dans lesquels circule un fluide échangeur de chaleur permettant de refroidir l'intérieur de la capacité. On peut utiliser un groupe frigorifique, disponible dans le commerce, équipé d'une pompe de circulation.

Pendant l'étape de chauffage, le fluide de refroidissement est, avantageusement, vidangé dans la capacité du groupe froid pour éviter son ébullition.

La capacité 100 est équipée d'un système de chauffage 400.

Le système de chauffage 400 peut être positionné à l'intérieur de la capacité.

De préférence, le système de chauffage 400 est externe. Le système de chauffage 400 est configuré pour chauffer l'intérieur de la capacité 100 jusqu'à des températures supérieures à 250°C et de préférence supérieures à 300°C de manière à vaporiser les goudrons condenser puis à les brûler.

Le système de chauffage 400 comprend des cordons électriques permettant de chauffer l'intérieur de la capacité 100. Les cordons électriques sont enroulés autour de la capacité 100.

Le système de chauffage 400 comporte, avantageusement, deux parties.

Une première partie est positionnée sur la paroi extérieure de la capacité 100, au niveau de la partie supérieure 102 de la capacité, i.e. entre l'entrée de syngas et le système de refroidissement 300. Avantageusement, cette première partie permet de maintenir la partie supérieure 102 de la capacité 100 à une température supérieure à celle de condensation des goudrons pour éviter que ceux-ci ne se condensent à l'entrée de la capacité et ne bouchent l'entrée 110 de la capacité 100. Par exemple, lors de l'étape c), un gradient de température dans la capacité allant de -20°C à 250°C peut être réalisé. La température est, par exemple, de 250°C au niveau de l'entrée de gaz 110 et de -20°C au niveau de la partie centrale de la capacité.

La deuxième partie du système de chauffage 400 est positionnée au niveau du système de refroidissement 300. Elle permet de chauffer la partie centrale 101 de la capacité 100 lors de l'étape de vaporisation et/ou de combustion des goudrons. Cette partie fonctionne en alternance avec le système de refroidissement.

La capacité 100 est également raccordée à un analyseur 500 de CO₂ permettant de déterminer la quantité de CO₂ via une ligne d'évacuation L₂. L'analyseur 500 de CO₂ peut également permettre de quantifier la quantité de CO. L'analyseur 500 peut être basé sur une technologie infrarouge non dispersive (NDIR), disponible dans le commerce. Une deuxième vanne V2 est disposée entre la capacité et l'analyseur de CO₂.

De préférence, la capacité 100 est également raccordée à un régulateur de débit massique 610 (RDM) permettant de fixer un débit stable. Eventuellement, un compteur peut être utilisé pour donner la valeur de la quantité de gaz passée.

Un compteur volumique de gaz 620 peut être positionné après l'analyseur 500.

De préférence, un piège froid 700, par exemple un piège barboteur rempli en isoPropanol à 0°C est positionné en sortie de la capacité 100, et avantageusement, entre la capacité 100 et l'analyseur 500 de CO₂. Il est, avantageusement, rempli d'isopropanol pour piéger les vapeurs résiduelles.

Avantageusement, le dispositif comprend, en outre, un capteur de pression 630 en sortie de la capacité 100 et/ou un thermomètre 640 permettant de mesurer la température à l'intérieur de la capacité 100.

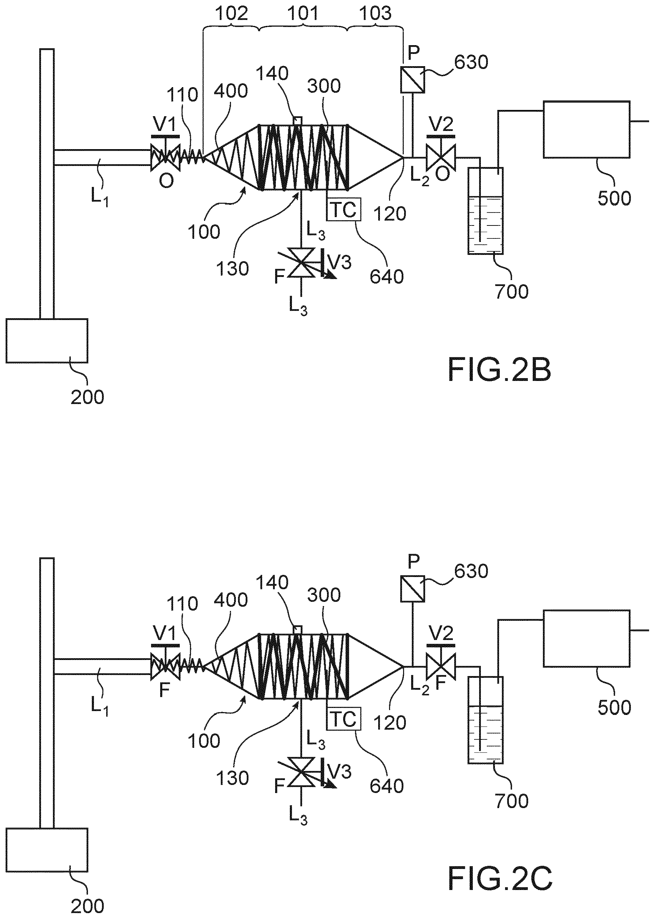

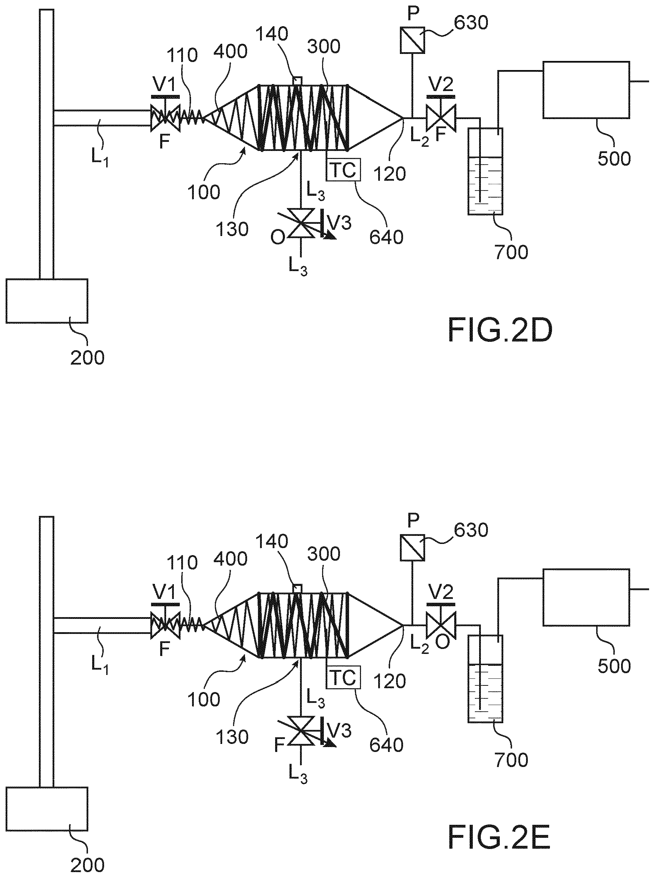

Nous allons maintenant décrire plus particulièrement un mode de réalisation avantageux du procédé d'évaluation de la quantité de goudrons dans un syngas. Le procédé comprend les étapes successives suivantes :
- fournir d'un dispositif tel que décrit précédemment,
- refroidir la capacité 100 à une température inférieure à -15°C, et de préférence inférieure à -20°C, avec le système de refroidissement 300 (figure 2A),
- ouvrir la première vanne V1 et la deuxième vanne V2, pour faire passer le gaz à travers la capacité 10), les goudrons se condensant dans la capacité 100, moyennant quoi on obtient des goudrons condensés (figure 2B),
- fermer la première vanne V1 et la deuxième vanne V2,
- chauffer la capacité 100, à une température supérieure à 250°C, avec le système de chauffage 400, de manière à vaporiser les goudrons condensés dans la capacité 100, moyennant quoi on forme des vapeurs de goudrons (figure 2C),
- ouvrir la troisième vanne V3, de manière à faire entrer du dioxygène dans la capacité 100 pour brûler les vapeurs de goudrons, moyennant quoi on forme des gaz de combustion (figure 2D),
- fermer la troisième vanne V3,
- ouvrir la deuxième vanne V2 pour évacuer les gaz de combustion par la sortie 120 de gaz jusqu'à l'appareil de mesure 500 et mesurer la quantité de CO₂ dans les gaz de combustion (figure 2E), puis
- déterminer la quantité de goudrons condensés puis brûlés à partir de la quantité de CO₂ mesurée.

Lors de la première phase, représentée sur la figure 2A, on refroidit la capacité 100 pour piéger les goudrons avec le système de refroidissement 300. Au moins l'intérieur de la partie centrale 101 de la capacité 100 est à une température inférieure à - 15°C, de préférence inférieure à -20°C, par exemple à une température allant de -20°C à - 40°C, et de préférence de -20°C à -30°C. La température choisie dépend de la nature du ou des goudrons à condenser. La température est mesure avec le thermomètre 640.

Simultanément, on peut chauffer la première partie du système de chauffage 400 pour éviter la condensation des goudrons dans la partie supérieure 101 de la capacité 100 et plus particulièrement au niveau de l'entrée 110 de la capacité 100.

Dans cette première phase, la première vanne V1, la deuxième vanne V2 et la troisième vanne V3 sont fermées.

Lors de la deuxième phase, on piège les goudrons à l'intérieur de la capacité 100 (figure 2B). La ligne de prélèvement L₁ de syngas est alimentée en syngas et la première vanne V1 et la deuxième vanne V2 sont ouvertes de manière à faire circuler le syngas à travers la capacité 100. Avantageusement, on fixe le débit. Le syngas circule dans la capacité 100 pendant une durée donnée. Le volume de gaz traversant la capacité est mesuré. Par exemple, le syngas circule dans la capacité 100 pendant 30min à 1L/min. Il est possible de déterminer la quantité de dioxyde de carbone initialement présente dans la syngas avant l'étape de combustion. On peut tenir compte de cette quantité quand on mesure la quantité de dioxyde de carbone dans les gaz de combustion.

Alternativement, entre l'étape de condensation et l'étape de combustion, il est possible de réaliser une purge de la capacité avec un gaz neutre pour que la mesure du dioxyde de carbone corresponde uniquement à celui issu des gaz de combustion. Par exemple, on établit une circulation d'azote via les vannes V3, V4 et V2, en régulant le débit via le régulateur de débit massique 610 et en mesurant le CO ou le CO₂ avec l'analyseur 500 jusqu'à avoir une concentration très faible (inférieure à 1%). On évitera de prolonger ce balayage pour ne pas entraîner de vapeurs de goudrons.

Lors d'une troisième phase, les goudrons sont vaporisés dans la capacité 100 (figure 2C). On ferme la première vanne V1 et la deuxième vanne V2. Le système de chauffage 400 est mis en route de manière à atteindre une température supérieure à la température de vaporisation des goudrons. On choisira par exemple une température supérieure à 250°C et de préférence supérieure à 300°C. Une température supérieure à 300°C facilitera la combustion. De préférence, la première partie du système de chauffage et la deuxième partie du système de chauffage fonctionnent. Les goudrons condensés se vaporisent dans le volume de la capacité 100.

Lors d'une quatrième phase, représentée sur la figure 2D, on ouvre la troisième vanne V3 pour réaliser la combustion des goudrons dans la capacité 100.

Par exemple, on injecte le dioxygène jusqu'à avoir une pression de 2 à 3 bars dans la capacité 100.

Lorsque la capacité est remplie du comburant, on active le système d'ignition 140.

Au contact du dioxygène et grâce à l'ignition, les vapeurs de goudrons brulent et produisent du CO₂ et, éventuellement du CO selon si la réaction de combustion est complète ou incomplète. Par exemple, pour le naphtalène la réaction de combustion est la suivante :

C₁₀H₈ + 12 O₂ → 10 CO₂ + 4 H₂O

De manière avantageuse, durant l'étape de combustion, on contrôle la pression avec le capteur 630 dans la capacité 100. Lorsque la pression est stable, on ferme la troisième vanne V3 pour arrêter l'alimentation en dioxygène.

Lors d'une cinquième phase, correspondant à la phase de mesure (figure 2E), on mesure le CO₂ produit lors de la combustion. La deuxième vanne V2 est ouverte. Le gaz, ou le mélange de gaz, résultant de la combustion des goudrons est évacué de la capacité 100 par la ligne L₂ jusqu'à l'analyseur 500. On intègre le signal de CO₂ obtenu, pour calculer la quantité de carbone qui était contenue dans les goudrons piégés dans la capacité 100. Il est alors possible de remonter à la quantité de goudrons initialement présente dans le syngas.

Dans les procédés de gazéification, il y a généralement un goudron majoritaire. Par exemple, si le naphtalène (C₁₀H₈) est le goudron majoritaire dans le syngas, une mole de Naphtalène donnera 10 moles de CO₂. Le résultat sera donc ramené à un « équivalent Naphtalène ».

Comme les goudrons ont des compositions variées, le résultat obtenu est donc un ordre de grandeur de la quantité de goudrons initialement présente dans le syngas.

Avantageusement, pour une combustion complète, on mesure et on intègre également le signal de CO.

De manière avantageuse, on peut réguler le débit. Le débit est, par exemple, régulé à 1L/min.

Durant cette phase, la pression dans la capacité redescend au fur et à mesure de l'évacuation des gaz de combustion. L'eau produite par la combustion est, avantageusement, condensée dans le piège 700.

Une fois l'analyse terminée, on ferme la deuxième vanne V2.

A titre illustratif et non limitatif, pour un piégeage de goudrons dans une capacité 100 à -20°C, avec une débit de gaz de 0,5Nl/min, un temps de séjour dans la capacité 100 à - 20°C de 25,4s, et une durée de piégeage de 10min, le tableau suivant donne les concentrations de CO₂ théoriques en fonction de différentes concentrations de goudrons dans le syngas.

| | | | |
|---|---|---|---|
| **Concentration en goudrons (g/Nm³)** | 0,1 | 1 | 10 |
| **Quantité de goudrons piégées (g)** | 0,0005 | 0,01 | 0,1 |

Pour un dégazage à 300°C, un débit de O₂ de 0,5Nl/min, un temps de séjour dans la capacité 100 à 300°C de 11,2s et un pic de CO₂ étalé sur 10min, le tableau suivant donne les masses de CO₂ théoriques produites lors de la combustion en fonction de différentes concentrations en CO₂.

| | | | |
|---|---|---|---|
| **CO₂ produit (g)** | 0,002 | 0,034 | 0,344 |
| **Concentration en CO₂ (ppm)** | 344 | 6875 | 68750 |

### REFERENCES

[1] Neeft et al. "Guideline for sampling and analysis of tar and particles in biomass producer gases", Report ECN-C-02-090 -2005.
[2] Dufour et al. "Comparison of two methods of measuring wood pyrolysis tar", Journal of Chromatography, 1164 (1-2) 2007, p240-247.
[3] "A comparison between on-line and off-line tar analysis methods applied to common reed pyrolysis", Patuzzi et al., Fuel 111 (2013) 689-695.
[4] Defoort et al. "A promising new on-line method of tar quantification by mass spectrometry during steam gasification of biomass", Biomass and Bioenergy 65 (2014) 64-71.
[5] Ahmadi et al. "Development of a PID based on-line tar measurement method - proof of concept", Fuel 113 (2013) 113-121.
[6] Sun et al. "Analysis of gas-phase polycyclic aromatic hydrocarbon mixture by laser induced fluorescence laser", Optics and Laser in Engineering 48 (2010) 1231-2137.
[7] Carpenter et al. "Quantitative measurement of biomass gasifier tars using a Molecular-Beam Mass Spectrometer: Comparison with Traditional Impinger Sampling", Energy & Fuels 21 (2007) 3036 -3043.
[8] Fendt et al. "On-line process Analysis of biomass Flash pyrolysis gases enabled by soft photoionization mass spectrometry", Energy &Fuels 26 (2012), 701-711.

## Revendications

1. Procédé d'évaluation de la quantité de goudrons dans un gaz, en particulier dans un syngas, comprenant les étapes successives suivantes :
a) fourniture d'un dispositif comprenant :
- une capacité (100) comprenant une entrée de gaz (110), raccordée à une ligne d'arrivée d'un gaz contenant des goudrons, une sortie de gaz (120), une entrée de dioxygène (130), et un dispositif d'ignition (140) pour amorcer la combustion de vapeurs de goudrons,
- un appareil de mesure (500) de CO₂, raccordé à la sortie de gaz (120),
- un système de refroidissement (300),
- un système de chauffage (400),
b) refroidissement de la capacité (100) à une température inférieure à - 15°C, et de préférence inférieure à -20°C, avec le système de refroidissement (300),
c) circulation du gaz à travers la capacité (100), depuis l'entrée de gaz (110) jusqu'à la sortie de gaz, les goudrons se condensant dans la capacité (100), moyennant quoi on obtient des goudrons condensés, puis arrêt de la circulation du gaz,
d) chauffage de la capacité (100), à une température supérieure à 250°C, et de préférence supérieure à 300°C, avec le système de chauffage (400), de manière à vaporiser les goudrons condensés dans la capacité 100, moyennant quoi on forme des vapeurs de goudrons,
e) introduction du dioxygène dans la capacité (100), par l'entrée de dioxygène, amorçage et combustion des vapeurs de goudrons, moyennant quoi on forme des gaz de combustion, puis arrêt de l'introduction de dioxygène,
f) évacuation des gaz de combustion par la sortie (120) de gaz jusqu'à l'appareil de mesure (500) et mesure la quantité de CO₂ dans les gaz de combustion, puis
g) détermination la quantité de goudrons condensés puis brûlés à partir de la quantité de CO₂ mesurée lors de l'étape f).

2. Procédé selon la revendication 1, **caractérisé en ce que** la capacité (100) comprend un solide poreux, par exemple une mousse métallique.

3. Procédé selon l'une des revendications 1 et 2, **caractérisé en ce qu'**un piège barboteur (700) est disposé entre la capacité (100) et l'appareil de mesure (500) de CO₂.

4. Procédé selon l'une quelconque des revendications 1 à 3, **caractérisé en ce que** le dispositif comprend en outre un régulateur de débit massique (610) pour contrôler le débit massique de gaz circulant à travers la capacité (100) lors de l'étape c).

5. Procédé selon l'une quelconque des revendications précédente, **caractérisé en ce que** le système de refroidissement (300) et/ou le système de chauffage (400) sont enroulés autour de la capacité.

6. Procédé selon l'une quelconque des revendications précédentes, **caractérisé en ce que** la capacité (100) comprend une partie centrale (101) tubulaire disposée entre une partie supérieure (102), de préférence de forme conique, et une partie inférieure (103).

7. Procédé selon la revendication précédente, **caractérisé en ce que** le système de refroidissement (300) est enroulé autour de la partie centrale (101) de la capacité (100) de manière à faire condenser les goudrons au centre de la capacité (100).

8. Procédé selon l'une des revendications 6 et 7, **caractérisé en ce que** le système de chauffage (400) comprend une première partie disposée autour de la partie supérieure (102) et une deuxième partie disposée autour de la partie centrale (101) de la capacité.

9. Dispositif comprenant :
- une capacité (100) comprenant une entrée de gaz (110), une sortie de gaz (120), une entrée de dioxygène (130),
- un appareil de mesure (500) de CO₂, raccordé à la sortie de gaz (120), et permettant de mesurer la quantité de CO₂,
**caractérisé en ce que** le dispositif comprend en outre :
- un système de refroidissement (300), configuré pour refroidir la capacité jusqu'à des températures inférieures à -15°C, et de préférence inférieures à - 20°C,
- un système de chauffage (400), configuré pour chauffer la capacité jusqu'à des températures supérieures à 250°C, et de préférence supérieures à 300°C,
et **en ce que** la capacité comprend un dispositif d'ignition (140) pour amorcer la combustion de vapeurs de goudrons.

## Patentansprüche

1. Verfahren zur Einschätzung der Menge von Teeren in einem Gas, insbesondere in einem Syngas, umfassend die folgenden sukzessiven Schritte:
a) Bereitstellen einer Vorrichtung, umfassend:
- eine Kapazität (100), umfassend einen Gaseintritt (110), der mit einer Zuleitung für ein Gas, das Teere enthält, verbunden ist, einen Gasaustritt (120), einen Disauerstoffeintritt (130) und eine Zündvorrichtung (140) zum Einleiten der Verbrennung von Teerdämpfen,
- ein COz-Messgerät (500), das mit dem Gasaustritt (120) verbunden ist,
- ein Kühlsystem (300),
- ein Heizsystem (400),
b) Kühlen der Kapazität (100) auf eine Temperatur, die niedriger als -15 °C und vorzugsweise niedriger als -20 °C ist, mit dem Kühlsystem (300),
c) Zirkulieren des Gases durch die Kapazität (100) von dem Gaseintritt (110) zu dem Gasaustritt, wobei die Teere in der Kapazität (100) kondensieren, wodurch kondensierte Teere erhalten werden, und dann Stoppen des Zirkulierens des Gases,
d) Erhitzen der Kapazität (100) auf eine Temperatur, die höher als 250 °C und vorzugsweise höher als 300 °C ist, mit dem Heizsystem (400), um die kondensierten Teere in der Kapazität 100 zu verdampfen, wodurch Teerdämpfe gebildet werden,
e) Einbringen des Disauerstoffs in die Kapazität (100) durch den Disauerstoffeintritt, Entzünden und Verbrennen von Teerdämpfen, wodurch Verbrennungsgase gebildet werden, und dann Stoppen des Einbringens von Disauerstoff,
f) Ableiten von Verbrennungsgasen durch den Gasaustritt (120) zum Messgerät (500) und Messen der CO₂-Menge in den Verbrennungsgasen und
g) Bestimmen der Menge von kondensierten und dann verbrannten Teeren aus während des Schritts f) gemessenen CO₂-Menge.

2. Verfahren nach Anspruch 1, **dadurch gekennzeichnet, dass** die Kapazität (100) einen porösen Feststoff, beispielsweise einen metallischen Schaum umfasst.

3. Verfahren nach einem der Ansprüche 1 und 2, **dadurch gekennzeichnet, dass** eine Gaswaschfalle (700) zwischen der Kapazität (100) und dem CO₂-Messgerät (500) angeordnet ist.

4. Verfahren nach einem der Ansprüche 1 bis 3, **dadurch gekennzeichnet, dass** die Vorrichtung weiterhin einen Massendurchflussregler (610) zum Steuern des Massendurchflusses von Gas, das während des Schritts c) durch die Kapazität (100) zirkuliert, umfasst.

5. Verfahren nach einem der vorhergehenden Ansprüche, **dadurch gekennzeichnet, dass** das Kühlsystem (300) und/oder das Heizsystem (400) um die Kapazität gewickelt sind.

6. Verfahren nach einem der vorhergehenden Ansprüche, **dadurch gekennzeichnet, dass** die Kapazität (100) einen rohrförmigen zentralen Teil (101) umfasst, der zwischen einem oberen Teil (102), vorzugsweise mit Konusform, und einem unteren Teil (103) angeordnet ist.

7. Verfahren nach dem vorhergehenden Anspruch, **dadurch gekennzeichnet, dass** das Kühlsystem (300) um den zentralen Teil (101) der Kapazität (100) gewickelt ist, um die Teere im Zentrum der Kapazität (100) kondensieren zu lassen.

8. Verfahren nach einem der Ansprüche 6 und 7, **dadurch gekennzeichnet, dass** das Heizsystem (400) einen ersten Teil, der um den oberen Teil (102) angeordnet ist, und einen zweiten Teil, der um den zentralen Teil (101) der Kapazität angeordnet ist, umfasst.

9. Vorrichtung, umfassend:
- eine Kapazität (100), umfassend einen Gaseintritt (110), einen Gasaustritt (120), einen Disauerstoffeintritt (130),
- ein COz-Messgerät (500), das mit dem Gasaustritt (120) verbunden ist und ein Messen der CO₂-Menge ermöglicht,
**dadurch gekennzeichnet, dass** die Vorrichtung weiterhin umfasst:
- ein Kühlsystem (300), das zum Kühlen der Kapazität auf Temperaturen, die niedriger als -15 °C und vorzugsweise niedriger als -20 °C sind, konfiguriert ist,
- ein Heizsystem (400), das zum Erhitzen der Kapazität auf Temperaturen, die höher als 250 °C und vorzugsweise höher als 300 °C sind, konfiguriert ist,
und dass die Kapazität eine Zündvorrichtung (140) zum Einleiten der Verbrennung von Teerdämpfen umfasst.

## Claims

1. A method for evaluating the amount of tars in a gas, in particular in a syngas, comprising the following successive steps:
a) providing a device comprising:
- a vessel (100) comprising a gas inlet (110), connected to an inlet line of a gas containing tars, a gas outlet (120), an oxygen inlet (130) and an ignition device (140) for initiating the combustion of tar vapors,
- a CO₂ measuring device (500), connected to the gas outlet (120),
- a cooling system (300),
- a heating system (400),
b) cooling the vessel (100) to a temperature below -15°C, and preferably below -20°C, with the cooling system (300),
c) circulating the gas through the vessel (100) from the gas inlet (110) to the gas outlet, the tars condensing in the vessel (100), whereby condensed tars are obtained, and then stopping the gas circulation,
d) heating the vessel (100) to a temperature above 250°C, preferably above 300°C, with the heating system (400), so as to vaporize the condensed tars in the vessel (100), whereby tar vapors are formed
e) introducing dioxygen into the container (100), through the dioxygen inlet, igniting and burning the tar vapors, thereby forming combustion gases, and then stopping the dioxygen inlet,
f) discharging the combustion gases through the gas outlet (120) to the measuring device (500) and measuring the amount of CO₂ in the combustion gases, then
g) determination of the amount of condensed and burned tars from the amount of the amount of CO₂ measured in step f).

2. The method according to claim 1, **characterized in that** the vessel (100) comprises a porous solid, for example a metal foam.

3. The method according to any of claims 1 and 2, **characterized in that** a bubbler trap (700) is arranged between the vessel (100) and the CO₂ measuring apparatus (500).

4. The method according to any one of claims 1 to 3, **characterized in that** the device further comprises a mass flow controller (610) for controlling the mass flow rate of gas flowing through the vessel (100) in step c).

5. The method according to any of the preceding claims, **characterized in that** the cooling system (300) and/or the heating system (400) are wrapped around the vessel.

6. Method according to any one of the preceding claims, **characterized in that** the vessel (100) comprises a tubular central portion (101) arranged between an upper portion (102), preferably conical in shape, and a lower portion (103).

7. A method according to the preceding claim, **characterized in that** the cooling system (300) is wrapped around the central portion (101) of the vessel (100) so as to condense the tars in the center of the vessel (100).

8. The method according to any of claims 6 and 7, **characterized in that** the heating system (400) comprises a first portion disposed around the top portion (102) and a second portion disposed around the central portion (101) of the vessel.

9. A device comprising:
- a vessel (100) comprising a gas inlet (110), a gas outlet (120), an dioxygen inlet (130),
- a CO₂ measuring device (500) connected to the gas outlet (120) for measuring the amount of CO₂
**characterized in that** the device further comprises:
- a cooling system (300), configured to cool the vessel to temperatures below -15°C, and preferably below 20°C,
- a heating system (400), configured to heat the vessel to temperatures above 250°C, and preferably above 300°C,
and **in that** the vessel comprises an ignition device (140) for initiating the combustion of tar vapors.
